Europäisches Patentamt

European Patent Office

Office européen des brevets

(11) Numéro de publication: **0 308 317**
**A1**

## (12) DEMANDE DE BREVET EUROPEEN

(21) Numéro de dépôt: 88402302.9

(51) Int. Cl.⁴: **A 61 K 9/68**

(22) Date de dépôt: 13.09.88

(30) Priorité: 14.09.87 FR 8712702

(43) Date de publication de la demande:
22.03.89 Bulletin 89/12

(84) Etats contractants désignés:
AT BE CH DE ES FR GB GR IT LI LU NL SE

(71) Demandeur: SANOFI
40, Avenue George V
F-75008 Paris (FR)

(72) Inventeur: Langlois, Christian
14 allée des Près Hauts
F-91370 Verrieres (FR)

(74) Mandataire: Le Guen, Gérard et al
CABINET LAVOIX 2, place d'Estienne d'Orves
F-75441 Paris Cédex 09 (FR)

Claims for the following Contracting States: ES + GR.

(54) **Composition anorexigène à mâcher et son procédé de préparation.**

(57) L'invention a pour objet une composition anorexigène à mâcher comprenant un noyau de gomme à mâcher contenant un agent édulcorant non calorique et une couche d'enrobage contenant un agent anorexigène.

EP 0 308 317 A1

Bundesdruckerei Berlin

**Description**

## COMPOSITION ANOREXIGENE A MACHER ET SON PROCEDE DE PREPARATION

La présente invention concerne des compositions pharmaceutiques à mâcher, anorexigènes, utiles dans le traitement des surcharges pondérales.

Les gommes à mâcher pharmaceutiques sont jusqu'à présent surtout utilisées pour le traitement des affections bucco-pharyngées, parce qu'elles assurent la présence prolongée du principe actif sur les muqueuses à traiter ; on a ainsi décrit dans FR-A-2386308 des gommes à mâcher pour la prévention des caries, contenant du fluor et dépourvues de sucres cariogènes et dans GB-A-2181646 des gommes à double action comprenant un principe actif dans le noyau de gomme, qui est libéré lentement, et un principe actif dans une couche entourant ce noyau, à libération d'autant plus rapide que la couche est plus hydrosoluble.

La présente invention vise à résoudre les problèmes spécifiques posés par une composition anorexigène à mâcher.

Les compositions anorexigènes selon l'invention comprennent un noyau de gomme à mâcher contenant un agent édulcorant non calorique et une couche d'enrobage contenant un agent anorexigène.

Alors qu'avec une forme pharmaceutique à mâcher on recherche généralement la libération prolongée du principe actif, dans la présente invention, grâce à la structure spécifique des compositions, la quasi-totalité du principe actif est libérée rapidement après la prise, qui a lieu de préférence au moment où l'envie de manger se manifeste ; ceci permet d'atteindre avec de faibles doses un niveau d'activité anorexigène satisfaisant lorsque le besoin s'en fait sentir, et cela sans risque de variations interindividuelles importantes, qui pourraient résulter d'intensités différentes de mastication, si le principe actif était noyé dans la masse de gomme.

De manière surprenante, chaque unité de composition peut ne contenir qu'une dose faible d'anorexigène, de 3 à 15 fois inférieure à la dose journalière généralement préconisée pour cet agent lorsqu'il est présenté sous une forme pharmaceutique classique : comprimé ou gélule, par exemple.

Comme les compositions selon l'invention ne contiennent pas de sucres riches en calories, tels que le saccharose ou le glucose, leur administration répétée dans la journée n'est pas contre-indiquée pour le régime basse-calories que doit suivre le patient ; toutefois, comme on a constaté qu'une mastication prolongée était importante pour qu'un effet anorexigène satisfaisant et soutenu se manifeste avec des doses de principe actif globalement inférieures à celles généralement administrées, la composition doit contenir une quantité suffisante d'agent édulcorant et éventuellement d'arômes pour que le patient lui trouve un goût agréable, sucré pendant une longue période.

De préférence, le noyau de gomme est séparé de la couche d'enrobage par un vernis qui facilite l'application de la couche d'enrobage et cette dernière est recouverte d'une couche isolante qui protège le principe actif ; la couche isolante peut être surmontée d'une couche de finition, selon une technique habituelle.

Le noyau peut être constitué notamment de gomme base et d'au moins un polyol édulcorant, qui donne au noyau sa texture en diluant et plastifiant la gomme base ; on peut éventuellement y ajouter un édulcorant synthétique pour renforcer le goût sucré, et des arômes alimentaires pour parfumer.

La gomme base peut être choisie parmi les gommes élastiques naturelles ou synthétiques et les élastomères alimentaires. Parmi celles-ci, on peut citer le caoutchouc naturel, le poly-isobutylène, les copolymères stryrène-butadiène et isoprène-isobutylène, etc... Elle peut constituer notamment de 30 à 40% en poids du noyau.

On choisit avantageusement l'édulcorant parmi le xylitol, le sorbitol, le mannitol et le maltitol, polyols qui sont couramment utilisés purs ou en mélange pour la fabrication des gommes à mâcher sans sucre. On préfère utiliser un mélange de xylitol et de sorbitol ou de mannitol, à raison de 10 à 20% de xylitol et de 30 à 50% de sorbitol en poids du noyau.

Le noyau peut contenir éventuellement un autre édulcorant tel que les saccharinates, l'aspartame, l'alitame ou l'acésulfame, les glycirrhizinates, les cyclamates, la thaumatine, le stévioside, les dihydrochalcones et les analogues de ces composés au pouvoir sucrant puissant ; il suffit d'utiliser, pour obtenir l'effet sucrant désiré, 0,1 à 3 mg d'un tel édulcorant dans un noyau de 100 mg à 1,5 g.

Les arômes, qui peuvent être mélangés à la gomme base, sont liquides ou en poudre ; on peut aussi utiliser un mélange de liquide et de poudre, la poudre assurant une aromatisation retardée mais prolongée. Ces arômes peuvent être des huiles ou des essences naturelles ou synthétiques, dérivées de fruits et de plantes, en solution ou absorbées sur des supports. Ils peuvent être utilisés en des quantités allant jusqu'à 10% en poids du noyau, suivant leur puissance aromatique et le goût recherché.

Les noyaux ont de préférence la forme de billes dont le diamètre est compris entre 5 et 15 mm, afin de faciliter les opérations d'enrobage ultérieures, mais ils pourraient aussi être de forme ovale, en galette, en coussinet, en oreiller ou autres.

La couche de vernis qui recouvre de préférence le noyau, isole ce dernier de la couche d'enrobage et facilite l'adhérence de cette couche à son support. Le vernis, dont l'épaisseur peut être comprise entre 0,005 mm et 0,02 mm, peut être constitué de gommes laque ou sandaraque, qui appliquées sur le noyau de façon classique en solution alcoolique de concentration comprise entre 2 et 10% (p/v). Le vernis peut être aussi à base d'éthylcellulose, d'un polyméthacrylate ou de leurs mélanges, et on peut introduire un plastifiant dans la couche de vernis, tel que l'huile de ricin, la paraffine ou un polyéthylène glycol.

Il faut souligner que les noyaux de gomme ne comportant pas de principe actif pharmaceutique, peuvent être préparés et éventuellement vernis, par les fabricants habituels de gommes à mâcher de confiserie, avant que ne soit appliquée la couche d'enrobage par des méthodes relevant des techniques galéniques classiques.

La couche de vernis ou le noyau sont recouverts de la couche d'enrobage dans laquelle est dispersé le principe actif pharmaceutique. Cette couche peut contenir, de façon classique un polyol, de préférence édulcorant, ainsi qu'éventuellement un agent d'adhérence, un plastifiant et un lubrifiant. Lorsque le principe actif est soluble dans le solvant servant au dépôt de la solution d'enrobage, généralement l'eau ou un mélange hydroalcoolique, il peut être introduit à la concentration voulue dans la solution d'enrobage, avant son dépôt sur le noyau par les techniques clas siques ; on peut aussi déposer le principe actif sous forme de solide divisé par poudrage du noyau sur lequel vient d'être déposé la solution d'enrobage. Cette opération de dépôt de la couche d'enrobage peut être effectuée en une seule fois ou résulter du montage successifs d'une série de couches. Il est également possible de fixer le principe actif sous forme de solide finement divisé directement sur les noyaux de gomme ramollis par simple mélange. Le ramollissement peut être obtenu en portant par exemple les noyaux à une température de 40 à 45°C.

Lorsque l'on dépose le principe actif sous forme de solide finement divisé il peut être constitué par un principe actif pulvérulent pur ou en mélange avec un diluant tel que le talc ; ou par un principe actif liquide absorbé sur une charge neutre, telle que le talc, le carbonate de calcium ou de magnésium ; ou encore par de microbilles, en général de diamètre inférieur à 0,3 mm, contenant le principe actif. Ces microbilles, ou microgranules, sont bien connues dans le domaine pharmaceutique où elles sont notamment utilisées pour préparer des formes à libération controlée. Dans le cas présent, elles peuvent servir à masquer le goût désagréable d'un principe actif, en diminuant sa concentration dans la bouche de telle sorte que le seuil de sensation d'amertume ne soit pas atteint. Les techniques permettant d'obtenir ces microbilles sont bien connues du spécialiste ; le principe actif peut être enrobé avec des polymères ou copolymères filmogènes naturels ou synthétique tels que gélatine, polysaccharides, dérivés de celluloses, résines acryliques, polyamides, polyesters, glycérides et autres par des procédés mettant en oeuvre par exemple la séparation de phases dont la coacervation, la polymérisation interfaciale ou in situ, le séchage par atomisation, la précipitation par refroidissement ou la technique des co-fondus.

Comme polyol utilisé dans la composition d'enrobage, on préfère le xylitol pour ses propriétés plastifiantes et édulcorantes mais on pourrait utiliser aussi le mannitol ; l'agent d'adhérence éventuel peut être choisi par exemple parmi les polyvinylpyrrolidones, les copolymères d'acétate de polyvinyle et de polyvinylpyrrolidone, les polyéthylène-glycols de masse moléculaire comprise entre 400 et 8 000 et les dérivés cellulosiques, tels que méthyl et éthyl-celluloses. En variante, au lieu d'utiliser un agent d'adhérence dans la couche d'enrobage on peut également déposer à chaud des silicones ou des glycérides saturés pulvérulents qui assurent à la fois une fonction d'adhérence et d'isolement.

La solution d'enrobage peut contenir jusqu'à 65% en poids de polyol et de 0,5 à 20% d'agent d'adhérence par rapport au poids de la solution, ainsi qu'un édulcorant puissant acalorique. La couche d'enrobage peut avoir une épaisseur comprise entre 0,1 et 5 mm ; elle sera fonction notamment de la quantité de principe actif que doit être fixée sur le noyau, qui peut varier entre 1 mg et 100 mg selon le principe actif.

Celui-ci peut être l'un quelconque des agents anorexigènes connus, du moment qu'il est chimiquement compatible avec les constituants de la couche d'enrobage, et que la dose unitaire nécessaire au traitement considéré n'est pas trop faible, pour garantir une homogénéité satisfaisante, ni évidemment très élevée, ce qui conduirait à des dragées très grosses, désagréables à mettre en bouche ; on peut citer, par exemple, les dérivés de phényléthylamine, comme le fenproporex, le clobenzorex, la phentermine, l'amfépramone, la fenfluramine.

La couche suivante isole la couche d'enrobage et le principe actif du milieu extérieur ; elle peut être réalisée de façon connue par additions successives d'une solution à base d'un polyol édulcorant, tel que le xylitol ou le sorbitol, et peut être aromatisée, colorée et sucrée avec un édulcorant acalorique. On peut aussi ajouter un plastifiant ou un adhésif dans le sirop de dépôt ainsi que des charges, tels que l'anhydride titanique, le carbonate de calcium, le talc. La couche finale résultant de l'application des différentes sous-couches peut avoir une épaisseur de 0,1 à 5 mm.

Eventuellement, les compositions de l'invention comportent aussi une couche de finition ou de lustrage, qui de façon classique est à base de glycérides synthétiques ou de cires, telle que la cire d'abeille et la cire de carnauba.

Les compositions anorexigènes à mâcher selon l'invention sont préparées par les techniques classiques de fabrication de dragées ou comprimés enrobés dans des turbines à dragéifier.

Dans ce qui suit, on décrit des exemples de réalisation de l'invention.

Exemple 1 : Dragée à mâcher, anorexigène , à base de chlorydrate de fenfluramine racémique.

a) Fabrication du noyau
On brasse en mélangeur de type pétrin, à bras en Z, pendant une à deux heures, à la température de 35 à 40°C, 3, 15 kg de gomme base, 1, 6 kg de xylitol, 3,54 kg de sorbitol avec 700 g d'arôme de menthe et 10 g de saccharinate de sodium. La masse obtenue est extrudée et les noyaux de gomme coupés à la dimension voulue.

Chaque noyau pèse environ 900 mg.

b) Vernissage du noyau
On prépare une solution contenant 6 g de polyméthacrylate granulé vendu par Rohm et Haas sous la référence Eudragit $^R$E100, dans un mélange d'isopropanol (44g) et d'acétone (50g). Les noyaux de gomme sont mis en rotation dans une turbine à dragéifier et le vernis d'isolement y est pulvérisé par petites quantités à raison de 0,5 l de vernis par 8 kg de noyaux. Le vernis est séché à l'air, à 25° C environ ; les noyaux vernis pèsent ainsi environ 902 mg.

c) Montage de la couche d'enrobage

On pulvérise par apports successifs sur les noyaux vernis en rotation dans une turbine à dragéifier, une solution préparée avec 410 g de xylitol, 5 g de sorbitol, 45 g de polyéthylène glycol 6000, 4 g de polyvinyl pyrrolidone, 65 g de talc et de l'eau pour 1 kg de solution, et après chaque apport on pulvérise sur les dragées en formation une poudre préparée avec 50 g de chlorhydrate de fenfluramine, 12,5 g de talc, 12,5 g de carbonate de calcium, 12,5 g d'éthycellulose et 12,5 g d'huile de ricin hydrogénée. Le noyau enrobé pèse environ 1200 mg et contient 5 mg de chlorhydrate de fenfluramine.

d) Application de la couche isolante.

Elle est effectuée en turbine à dragéifier par pulvérisation d'un sirop préparé avec 410 g de xylitol, 30g de polyéthylène glycol 6000, 4 g de polyvinylpyrrolidone, 30g d'anhyride titanique, 2,5 g d'arôme menthe et de la quantité d'eau nécessaire pour faire 1 kg de sirop.

Les noyaux dragéifiés pèsent à la fin de cette étape environ 1790 mg

e) Finition de la dragée

On disperse dans 93,5g d'acétate d'éthyle, 6,5 g de cire blanche et l'on agite, pentant une heure environ, jusqu'à obtention d'une suspension qui est appliquée sur les noyaux enrobés. Après séchage à l'air ambiant, on peut faire briller la couche de finition en tonneau à lisser.

L'utilisation de ces dragées à mâcher dans le traitement des surcharges pondérales est particulièrement avantageux, puisqu'à côté de leur action anorexigène due à la présence de la fenfluramine, cette forme pharmaceutique permet la maintenance de l'activité masticatoire du malade, satisfaisant certaines des habitudes alimentaires, sans apport caloriques, étant donné l'absence de sucres.

Exemple 2 : Dragées à mâcher à base de chlorhydrate de D-fenfluramine

On prépare ces dragées en appliquant le procédé décrit à l'exemple 1 mais en fixant à l'étape C 250 ou 500 g de microgranules contenant le principe actif à la place du chlorhydrate de fenfluramine racémique.

Les microgranules sont composés de 10g de chlorydrate de D-fenfluramine pour 100 g d'un mélange de glycérides (50%), d'huile de ricin hydrogénée (25%) et cire de carnauba (25%).

**Revendications**

1. Composition anorexigène à mâcher comprenant un noyau de gomme à mâcher contenant un agent édulcorant non calorique et une couche d'enrobage contenant un agent anorexigène.

2. Composition selon la revendication 1, caractérisée en ce que le noyau est constitué de gomme base et d'au moins un polyol édulcorant et plastifiant.

3. Composition selon la revendication 2, caractérisée en ce que le polyol est choisi parmi le xylitol, le sorbitol, le mannitol et le maltitol.

4. Composition selon l'une des revendications 2 et 3, caractérisée en ce que le noyau contient, en outre, un édulcorant synthétique puissant.

5. Composition selon l'une des revendications précédentes, caractérisée en ce que la couche d'enrobage contient, en outre, un polyol.

6. Composition selon l'une la revendication 5, caractérisée en ce que le polyol de la couche d'enrobage est choisi parmi le xylitol et le mannitol.

7. Composition selon l'une des revendications précédentes, caractérisée en ce que la couche d'enrobage est isolée du noyau par un vernis.

8. Composition selon l'une des revendications précédentes, caractérisée en ce que la composition est revêtue d'une couche isolante.

9. Composition selon l'une des revendications précédentes, caractérisée en ce que l'agent anorexigène est la fenfluramine.

10. Procédé de préparation d'une composition de l'une quelconque des revendications 1 à 9, caractérisé en ce que l'on dépose sur un noyau de gomme à mâcher contenant un agent édulcorant non calorique, une couche d'enrobage contenant un agent anorexigène.

11. Procédé selon la revendication 10, caractérisé en ce que l'agent anorexigène est introduit dans la solution d'enrobage avant son dépôt sur le noyau.

**Revendication pour les Etats contractants suivants: ES, GR.**

1. Procédé de préparation d'une composition anorrexigène à mâcher, caractérisé en ce que l'on dépose sur un noyau de gomme à mâcher contenant un agent édulcorant non calorique, une couche d'enrobage contenant un agent anorexigène.

2. Procédé selon la revendication 1, caractérisée en ce que le noyau est constitué de gomme base et d'au moins un polyol édulcorant et plastifiant.

3. Procédé selon la revendication 2, caractérisé en ce que le polyol est choisi parmi le xylitol, le sorbitol, le mannitol et le maltitol.

4. Procédé selon l'une des revendications 2 et 3, caractérisé en ce que le noyau contient, en outre, un édulcorant synthétique puissant.

5. Procédé selon l'une des revendications précédentes, caractérisé en ce que la couche d'enrobage contient, en outre, un polyol.

6. Procédé selon la revendication 5, caractérisé en ce que le polyol de la couche d'enrobage est choisi parmi le xylitol et le mannitol.

7. Procédé selon l'une des revendications précédentes, caractérisé en ce que la couche d'enrobage est isolée du noyau par un vernis.

8. Procédé selon l'une des revendications précédentes, caractérisé en ce que la composition est revêtue d'une couche isolante.

9. Procédé selon l'une des revendications précédentes, caractérisé en ce que le principe actif est la fenfluramine.

10. Procédé selon la revendication 1, caractérisé en ce que l'agent anorexigène est introduit dans la solution d'enrobage avant son dépôt sur le noyau.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X,Y | US-A-2 262 087 (K.A. BARTLETT)<br>* En entier *<br>--- | 1-11 | A 61 K 9/68 |
| Y | GB-A-2 079 129 (LIFE SAVERS INC.)<br>* Revendications *<br>--- | 2-11 | |
| Y | DE-A-3 414 743 (STADELMAYR)<br>* Page 5, lignes 15-18 *<br>--- | 1-11 | |
| A,D | FR-A-2 386 308 (J.J. GOUPIL)<br>* Revendications *<br>--- | 1-11 | |
| A,D | GB-A-2 181 646 (D. MORRIS)<br>* Revendications *<br>----- | 1-11 | |

DOMAINES TECHNIQUES
RECHERCHES (Int. Cl.4)

A 61 K 9/00

Le présent rapport a été établi pour toutes les revendications

| Lieu de la recherche | Date d'achèvement de la recherche | Examinateur |
|---|---|---|
| LA HAYE | 13-12-1988 | GERLI P.F.M. |

CATEGORIE DES DOCUMENTS CITES

X : particulièrement pertinent à lui seul
Y : particulièrement pertinent en combinaison avec un
autre document de la même catégorie
A : arrière-plan technologique
O : divulgation non-écrite
P : document intercalaire

T : théorie ou principe à la base de l'invention
E : document de brevet antérieur, mais publié à la
date de dépôt ou après cette date
D : cité dans la demande
L : cité pour d'autres raisons
........................................................................
& : membre de la même famille, document correspondant

EPO FORM 1503 03.82 (P0402)